# EUROPEAN PATENT APPLICATION

(11) **EP 1 673 983 A1**
(43) Date of publication of application: **28.06.2006**
(21) Application number: 05027599.9
(22) Date of filing: 16.12.2005
(51) Int. Cl.: A23K 1/17

(54) **A methane production reducer, and feed composition for ruminant**

(30) Priority: 17.12.2004 JP 2004366914
(71) Applicant: Ajinomoto Co., Inc., Tokyo 104-8315 (JP); Obihiro University of Agriculture and Veterinary, Obihiro-shi Hokkaido 080-8555 (JP)
(72) Inventor: Uehara, Akinori, Kawasaki-shi Kanagawa 210-8681 (JP); Toride, Yasuhiko, Kawasaki-shi Kanagawa 210-8681 (JP); Takahashi, Junichi, Obihiro-shi Hokkaido 080-8555 (JP)
(74) Representative: HOFFMANN EITLE

(57) **Abstract**

The present invention relates to a methane production reducer and a feed composition for ruminants, and more particularly, to a methane production reducer for ruminants comprising protease-resistant bacteriocin isolated from lactic acid bacteria, and/or a feed composition for ruminants containing this methane production reducer, and/or a method of improving the growth of ruminant by administering the feed composition.

## Description

### [Background of the invention]

The present invention relates to a methane production reducer and a feed composition for ruminants, and more particularly, to a methane production reducer for ruminants comprising protease-resistant bacteriocin isolated from lactic acid bacteria, and/or a feed composition for ruminants containing this methane production reducer, and/or a method of improving the growth of ruminant by administering the feed composition.

The methane produced in the rumen of a ruminant not only constitutes inefficient use of the energy in feed, but is also a greenhouse gas contributing to global warming. Thus, it is highly important to reduce methane production.

Fermentation is conducted in the rumen by a variety of microbes, resulting in various metabolites. One of these, methane, is thought to be produced by methane producing bacteria. The methane producing bacteria in the rumen are hydrogen-assimilating bacteria that utilize hydrogen to reduce carbon dioxide, generating methane. Accordingly, the presence of a more powerful reduction reaction inhibits methane production (WO 96/39860 and JP2003-88301A).

The administration of ionophores such as monensin and iberin to ruminants is a known method of inhibiting methane production in the rumen. The supplying of cysteine (JP 7-322828A) and the supplying of fumaric acid (JP2003-88301A and JP7-322828A) are known methods that have received attention in the adjustment of reduction capability in the rumen. The addition of nitrates has also been reported to be effective, but since toxic nitrites accumulate in the rumen, the addition of nitrates is known to cause nitrite poisoning of ruminants.

The antibiotic compound bacteriocin that is produced by microorganism has also been investigated. Although the sole use of nisin produced by lactic acid bacteria has been reported (WO 99/41978), nisin is readily degraded by protease in the rumen and thus does not have a lasting effect. Further, the appearance of nisin-resistant bacteria has also been reported (James B. Russell et al., Current Microbiology, Vol. 35, p. 90-96, (1997).). Still further, feed efficiency can be improved by the combined use of preparations of sorbic acid and bacteriocin (JP2002-262780A). However, methane production inhibition when these are added to the feed of ruminants is unclear, and as is the case for nisin, a prolonged effect cannot be anticipated for conventional protease-sensitive bacteriocin.

### [Summary of the invention]

Accordingly, object of the present invention include providing a methane production reducer and feed composition which reduce the production of methane in the rumen of ruminant, enhances feed efficiency are disclosed. And a method of improving the growth of ruminant by administering this feed composition are disclosed.

The present inventors conducted extensive research into solving the above-stated problems, resulting in the discovery that the administration of a methane production-reducer comprising protease-resistant bacteriocin isolated from lactic acid bacteria into the rumen (fluid) of a ruminant permitted the suppression of methane production in the rumen; the present invention was devised on that basis.

It is an object of the present invention to provide to a methane production reducer for ruminants comprising protease-resistant bacteriocin isolated a lactic acid bacterium.
It is a further object of the present invention to provide a methane production reducer comprising isolated lactic acid bacteria culture solution and/or lactic acid culture supernatant.
It is a further object of the present invention to provide the methane production reducer, wherein said reducer is formulated for administration to ruminants.
It is a further object of the present invention to provide the methane production reducer wherein in that said lactic acid bacterium is one or more lactic acid bacteria selected from the group consisting of the bacterium belonging to the genus Lactobacillus, Weissella, Pediococcus, and Leuconostoc.
It is a further object of the present invention to provide the methane production reducer wherein the genus lactobacillus is selected from the group consisting of Lactobacillus plantarum, Lactobacillus salivarius, and/or Lactobacillus pentosus.
It is a further object of the present invention to provide the methane production reducer, wherein said lactic acid bacterium of the genus Weissella is selected from the group consisting of Weissella sp. FERM ABP-10474, Weissella cibaria, Weissella confusa, Weissella hellenica, Weissella kandleri, Weissella minor, Weissella paramesenteroides, and/or Weissella thailandensis.
It is a further object of the present invention to provide the methane production reducer wherein f said lactic acid bacterium of the genus Pediococcus is Pediococcus pentosaceus.
It is a further object of the present invention to provide the methane production reducer wherein the genus Leuconostoc is selected from the group consisting of Leuconostoc citreum, Leuconostoc pseudomesenteroides, Leuconostoc argentinum, Leuconostoc carnosum, and/or Leuconostoc mesenteroides.
It is a further object of the present invention to provide a feed composition for ruminants comprising the methane production reducer.
It is a further object of the present invention to provide method of improving the growth of ruminant by administering the above feed composition said ruminant.
It is a further object of the present invention to provide a use of a methane production reducer for ruminants comprising protease-resistant bacteriocin isolated from a lactic acid bacterium. [0009] Administration of the methane production-inhibiting agent of the present invention significantly inhibits the generation of methane in the rumina of ruminants, improves the energy efficiency of feeds, and as a result, enhances the development of ruminants. Further, by reducing the generation of methane, a greenhouse gas, a contribution is made to environmental issues such as global warming.

### [Detailed description of the invention]

Hereafter, the present invention will be described in detail below.

The methane production reducer for ruminants of the present invention is characterized by comprising protease-resistant bacteriocin isolated lactic acid bacteria. The "ruminants" referred to in the present invention are mammals belonging to the suborder of ruminants of the order Artiodactyla, having stomachs divided into three or four compartments, and ruminating their food. Examples are cattle and sheep.

Generally, "bacteriocin" refers to an antibiotic protein substance (Klaenhammer, T.R., Biochemie 60(3): 337-349 (1988)). However, the "protease-resistant bacteriocin" of the present invention refers to bacteriocins that are not degraded by protein degrading enzymes (proteases), in contrast to conventional bacteriocins such as nisin. The bacteriocins of the present invention are not degraded by proteases present in the rumen of ruminants or by proteases derived from rumen bacterium. "Proteases derived from rumen bacterium" means, for example, protease derived from *Butyrivibrio fibrisolvens,* and proteases derived from *Ruminobacter amylophilus, Butyrivibrio fibrosolvens* [sic], and *Prevotella ruminicola. (The Nutritional Physiology of Ruminants* (Nobunkyo), Yasuyuki SASAKI, or Wallace R. J., Onodera, R., and Cotta, M.A. (1997) pp. 283-328, Academic and Professional.)

In addition to being resistance to proteases present in the rumen and proteases derived from ruminal bacterium, the protease-resistant bacteriocins isolated lactic acid bacteria that is employed in the present invention also is resistant to, and is not degraded by, proteases derived from the genus Aspergillus which are employed in brewing and fermentation, as well as proteases derived from meats, which are employed in food processing.
Examples of these proteases are "Umamizyme G" (Amano Enzymes Inc), a protease of the genus Aspergillus that is employed in brewing and fermentation, and cathepsin, a protease derived from meat that is employed in food processing. These are representative of the proteases to which the bacteriocin of the present invention is resistant; however, proteases to which the bacteriocin of the present invention is resistant are not limited thereto.

The protease-resistant bacteriocin of the present invention is produced by lactic acid bacteria and is highly safe. This protease-resistant bacteriocin has an antibiotic action on rumen bacterium. Thus, when this protease-resistant bacteriocin, or a culture solution or culture supernatant containing it, is administered to the rumen (as a feed composition), the bacteriocin remains present without being degraded by protease, suppressing the methane-generating effect of methane-producing microbes in the rumen. Further, this bacteriocin is derived from lactic acid bacteria, and is safer than conventional chemically synthesized compounds even when consumed by the ruminants in large quantities. It is thus also desirable from the aspect of the health of the ruminants.

This protease-resistant bacteriocin can be efficiently manufactured by culturing the lactic acid bacteria according to the example below.

The lactic acid bacteria producing the protease-resistant bacteriocin that are employed in the present invention have been isolated from fermented foods and the like. Any lactic acid bacteria with antibiotic activity that can be detected by the screening method described below may be employed, even when separated from something other than fermented foods or the like.

The lactic acid bacteria employed in the present invention preferably belong to the genus Lactobacillus, Weissella, Pediococcus, or Leuconostoc. In particular, preferable examples of lactic acid bacteria belonging to the genus Lactobacillus are: *Lactobacillus plantarum, Lactobacillus salivarius,* and *Lactobacillus pentosus.* Preferable examples belonging to the genus Weissella are: *Weissella sp.* FERM ABP-10474, *Weissella cibaria, Weissella confusa, Weissella hellenica, Weissella kandleri, Weissella minor, Weissella paramesenteroides,* and *Weissella thailandensis.* A preferable example belonging to the genus Pediococcus is *Pediococcus pentosaceus.* And preferable examples belonging to the genus Leuconostoc are: *Leuconostoc citreum, Leuconostoc pseudomesenteroides, Leuconostoc argentinum, Leuconostoc carnosum,* and *Leuconostoc mesenteroides.*

Of the above lactic acid bacteria b, the following are particularly preferable for use in the present invention: *Lactobacillus plantarum* strain JCM1149, *Lactobacillus salivarius* strain JCM1231, and *Lactobacillus pentosus* strain JCM1558; *Pediococcus pentosaceus* strains JCM5885 and JCM5890, *Weissella sp.* FERM ABP-10474, *Weissella cibaria* strain JCM12495, *Weissella confusa* strain JCM 1093, *Weissella hellenica* strain JCM10103, *Weissella kandleri* strain JCM5817, *Weissella minor* strain JCM1168, *Weissella paramesenteroides* strain JCM9890, and *Weissella thailandensis* strain JCM10694, and *Leuconostoc citreum* strain JCM9696, *Leuconostoc pseudomesenteroides* strain JCM11945, *Leuconostoc argentinum* strain JCM11052, *Leuconostoc carnosum* strain JCM9695, and *Leuconostoc mesenteroides* strain JCM6124. The bacterial strains referenced by JCM depository numbers are stored "Japan Collection of Microorganisms" of the Riken Bioresource Center (an Independent Administrative Institution), 2-1 Hirozawa, Wako, Saitama Prefecture, Japan. *Weissella sp.* FERM ABP-10474 was deposited as depository number FERM ABP-10474 at the "International Patent Organism Depositary" of the National Institute of Advanced Industrial Science and Technology (an Independent Administrative Institution), Central 6, 1-1-1 Tsukuba East, Ibaraki Prefecture, Japan, on October 31,2003).

Whether or not a given lactic acid bacterium produces the protease-resistant bacteriocin (sometimes abbreviated to "PRB" below) of the present invention can be determined by the following method, for example. That is, in the following method, growth inhibition zones of an indicator strain are formed when PRB is being produced in a culture of lactic acid bacteria.
(1) A lactic acid bacterium culture solution is prepared by a typical culture method for lactic acid bacteria (or by a culture method in which lactic acid bacterium of this invention is separated). The culture solution of the lactic acid bacterium is adjusted to pH 5.5 to 6.0 with NaOH, separated by centrifugation at 12,000 rpm x 10 min, and filtered through 0.45 µm cellulose acetate with a Disposable Syringe Filter Unit (ADVANTEC "Dismic-25cs) to obtain a sample. When antibiotic activity is low, fourfold concentration is conducted under reduced pressure at room temperature. When necessary, tenfold concentration may be conducted.
*(2) Listeria innocua* ATCC33090T, *Bacillus circulans* JCM2504T, *Bacillus coagulans* JCM2257, *Micrococcus luteus* IF012708, *Bacillus subtilis* JCM1465T, *Bacillus subtilis* IAM1381, *Lactococcus lactis* sub sp. Lactis ATCC19435, *Enterococcus faecium* JCM5804T, *Enterococcus faecium* JCM5803T, *Lactobacillus plantarum* ATCC14917TL, and *Lactobacillus sakei* JCM1157T are employed as the indicator strain. Antibiotic activity is measured by the spot-on-lawn method, described further below, or the viable bacteria count method, and the indicator strain which exhibiting the strongest antibiotic activity is selected.
(3) Aspergillus-derived protease ("Umamizyme G" or the like made by Amano Enzymes (K.K.)) is employed as the enzyme.
(4) A 10 to 100 unit/mL quantity of the enzyme described in (3) is added to the sample of (1) and a reaction is conducted by maintaining the mixture at 30°C for one or more hours.
(5) The indicator strain that exhibited the greatest antibiotic activity in (2) is plated, 0.01 mL of the sample which has been treated with the enzyme of (4) is added dropwise to a medium in which the indicator strain will proliferate, such as MRS, and culturing is conducted for 20 to 24 hours at the optimum temperature for growth of the indicator strain (37°C for *Listeria innocua, Bacillus coagulans, Enterococcus faecium,* and *Pediococcus pentosaceus,* 30°C for the others). Subsequently, growth inhibition zones of the indicator strain are confirmed. Here, the bacterial strains for which JCM depository numbers are given are in storage at the "Japan Collection of Microorganisms" of the Riken Bioresource Center (an Independent Administrative Institution), 2-1 Hirozawa, Wako, Saitama Prefecture, Japan. Bacterial strains for which ATCC depository numbers are given can be obtained by registration number from the American Type Culture Collection, for example. The registration number corresponding to each bacterial strain is recorded in the catalog of the American Type Culture Collection. Bacterial strains for which LAM depository numbers are given are stored in the Bioresource Research Field IAM Culture Collection of the Molecular and Cellular Biology Institute of Tokyo University and are available by registration number. The registration number corresponding to each bacterial strain is recorded in the IAM Catalog of Strains, 3^{rd} Edition, 2004.

The methane production reducer of the present invention, comprising protease-resistant bacteriocin, may include the lactic acid bacterium culture solution which produces the protease-resistant bacteriocin as is, and/or may include the dried bacterial product of such a culture solution, or may include the culture supernatant. Bacteriocin obtained by the separation and purification of any of these may also be included in the composition of the present invention. A suitable excipient or the like, as described further below, may also be added to the composition of the present invention. In brief, an agent exhibiting PRB activity derived from lactic acid bacteria suffices as a component of the composition of the present invention. In passing, (the activity) of PRB produced by protease-resistant bacteriocin produced from lactic acid bacteria is present intracellularly, extracellularly.

Protease-resistant bacteriocin produced from a culture solution of lactic acid bacteria can be separated and purified as necessary according to the methods commonly employed in the field. Specifically, the bacteriocin can be produced by obtaining a fraction which has the protease-resistant bacteriocin activity and conducting ammonium sulfate precipitation, column chromatography, ethanol precipitation, or the like. The lactic acid bacterium employed in the present invention can be cultured using medium components suited to the bacterial strain employed and production of protease-resistant bacteriocin. The use of a culture solution that has been suitably concentrated permits more efficient further processing.

Lactic acid bacteria can be cultured by typical methods such as those shown below.

A carbon source may be present in the medium employed for the present invention, starch sugar solution, or food-use glucose. A nitrogen source in may also be present in the medium employed for present invention and may include protein concentrate hydrolysis products, corn peptides, soy peptides, commercial flavoring solution materials, low-end distilled spirit lees, or food-use enzyme extracts. Additionally, various organic and inorganic products, and items containing such products, that are required for the growth of lactic acid bacteria and enzyme production, such as phosphates, magnesium salt, calcium salt, manganese salt, and other salts; vitamins; and yeast extract, may be suitably added to the medium. The culturing temperature and period may be set according to those typical in common lactic acid bacteria culturing methods; for example, in a stationary culture, the temperature and time period for culture may be set to 30 to 37°C and 12 to 36 hours, respectively .

In the present invention, confirmation of the reductive effect of administration of the protease-resistant bacteriocin of the present invention on methane production in the rumen is possible with an artificial rumen system (T. Hino et al., J. Gen. Appl. Microbiol., 39, 35-45 (1993)) or by actual *in vivo* oral administration to ruminants.

The methane production reducer for ruminants of the present invention may be employed in various forms. Examples are powders, granules, and tablets. Excipients, fillers, and the like may be suitably added as needed. When a PRB-producing lactic acid bacterium culture solution is employed as the composition of the present invention, the proportion of the lactic acid bacterium of the present invention in the composition may be determined relative to the quantity of bacteria responsible for ruminant. When the protease-resistant bacteriocin is of high purity or the specific activity is high, a small quantity is administered, and when the medium itself is being administered or the specific activity is low, a high ratio is administered.

The time of administration of the methane production reducer or composition of the present invention is not specifically limited so long as the inhibitive effect on methane production of the present invention is exhibited. As long as the feed is retained in the rumen, administration at any time is possible. However, since the methane production reducer is preferably present in the rumen at about the time methane is produced, the methane production-reducer or composition is preferably administered immediately before or simultaneously with feed administration. Blending the composition into the feed permits particularly efficient administration.

The dose of methane production reducer of the present invention that is administered is not specifically limited so long as the inhibitive effect on methane production of the present invention is exhibited. For example, roughly 10 percent of the amount of rumen juice in the rumen may be administered. Specifically, administration may be based on a calculation of the average rumen juice level per unit of body weight. Roughly 20 percent of the body weight of a ruminant is thought to be in the form of rumen juice ("New Livestock Handbook", Kodansha, 1995). Suitable adjustment is made based on the animal receiving administration and the lactic acid bacterium employed in pre-testing.

The feed composition for ruminants of the present invention is a feed composition to which the above-described methane production reducer is added. The blending ratio of the methane production reducer in the feed composition is normally 0.1 to 10 weight percent, preferably 1 to 5 weight percent, based on the culture solution.

The feed composition for ruminants is not specifically limited; a commercial product can be used as is, or suitable silage or hay may be added to a commercial product as needed. An example of a bovine feed composition is 40 percent corn silage, 14 percent Sudan grass hay or alfalfa hay cubes, and 46 percent commercial feed blend. As an example, a commercial feed blend may contains not less than 16 percent of crude protein or not less than 71 percent of digestible total nutrients. As a further example, there are sheep feed compositions comprising 85 percent timothy hay and 15 percent commercial feed blend.

In the present invention, feed compositions into which the methane production reducer has been blended may be freely fed to ruminants. They may also be fed for extended periods. Doing this inhibits methane production in ruminants and thus enhances the growth of ruminants.

### [Examples]

The present invention is specifically described below through the following nonlimiting Examples.

### <Reference Example 1 >

The method of screening lactic acid bacteria producing protease-resistant bacteriocin, an important point in the present invention, will be described based on the example of separation from the fermented food matsoon.

To separate the lactic acid bacteria, 0.5 percent of fermented milk matsoon (a type of fermented food) samples were added to liquid media which
permit the growth of lactic acid bacteria, such as MRS medium (Table 1 below) and M17 medium (Table 2 below). The samples were cultured at 30 to 37°C preculturing). Based on the properties of the bacterial strains and culturing conditions, culturing was conducted for one, five, or ten days, respectively. Upon completion of culturing, the bacteria were cultured on the above-described agar (1.2 percent) medium containing 0.5 percent calcium carbonate and the lactic acid bacteria colonies that grew were collected.

[Table 1]

**Table 1**

| MRS medium composition (Merck) | |
|---|---|
| Peptone | 10.0 g/L |
| Lab-Lemco' Powder | 8.0 g/L |
| Yeast extract | 4.0 g/L |
| Glucose | 20.0 g/L |
| Tween 80 | 1.0 g/L |
| Dipotassium hydrogenphosphate | 2.0 g/L |
| Sodium acetate | 5.0 g/L |
| Ammonium citrate | 2.0 g/L |
| Magnesium sulfate 7 hydrate | 0.20 g/L |

[Table 2]

**Table 2**

| M17 medium composition (Merck) | |
|---|---|
| Soymeal derived peptone | 5.0 g/L |
| Meat derived peptone | 2.5 g/L |
| Casein derived peptone | 2.5 g/L |
| Yeast extract | 2.5 g/L |
| Meat extract | 5.0 g/L |
| D(+) lactose | 5.0 g/L |
| Ascorbic acid | 0.5 g/L |
| β-glycerophosphoric acid sodium | 19.0 g/L |
| Magnesium sulfate | 0.25 g/L |

The collected lactic acid bacteria that were collected were similarly cultured in the liquid medium and under the culture conditions as set forth above (the main culture). Next, the lactic acid bacteria were inoculated onto MRS agar medium plates to which pre-filtered "Umamizyme G" (Amano Enzymes (K.K.)), a protease derived from Aspergillus oryzae, had been added, and cultured for 24 hours at 30°C. These plates were then layered with Lactobacilli AOAC medium (Table 3 below) mixed with indicator bacteria and the bacteria were cultured for 24 hours on these plates at 30°C, which resulted in growth inhibition zones of indicator bacteria to form.

[Table 3]

**Table 3: Lactobacilli AOAC medium composition**

| Lactobacilli AOAC medium composition (Difco) | |
|---|---|
| Peptonized milk | 15.0 g/L |
| Yeast extract | 5.0 g/L |
| Dextrose | 10.0 g/L |
| Tomato juice | 5.00 g/L |
| Monopotassium dihydrogen phosphate | 2.0 g/L |
| Polysorbate 80 | 1.0 g/L |

The protease can also be added by the following methods instead of by addition to an agar medium. Examples of such methods are: 1) mixing the protease with the indicator strains; 2) coating the protease on agar medium; 3) adding the protease in the course of cultivating lactic acid bacteria colonies (in this process, the protease can be added at the start of the culture, during the culture, or once the culture has been completed); and 4) after cultivating lactic acid bacteria colonies, eliminating the bacterial mass or killing the bacteria in the culture solution, adding to plates into which the indicator strains have been admixed a suitable quantity of sample to which protease has been added, and confirming the formation of inhibition zones. Methods 1) to 4) above are given by way of example and are not limitations. Nor is the protease limited to "Umamizyme G".

Next, protease-resistant bacteriocin activity was evaluated by antibiotic spectral analysis. The antibiotic spectrum was examined by the spot-on-lawn method, in which the culture solution supernatant of a lactic acid bacteria exhibiting antibiotic activity was sequentially diluted and spotted on an antibiotic activity plate, described further below.

First, antibiotic activity samples were prepared. The culture solution of a strain having antibiotic activity collected by the above-described method was centrifugally separated for 10 min at 10,000 rpm, yielding the culture supernatant. The culture supernatant was then passed through a filter to obtain a sterile sample. The sample was then diluted in twofold steps to prepare a 2¹¹ diluted solution. When the activity was low, as needed, reduced pressure concentration was conducted in twofold steps at room temperature to prepare a 2⁻³ diluted solution.

Next, the mixed indicator strain was cultured on an antibiotic activity plate. The indicator strain recorded in Table 4 below were cultured on TSBYE medium (Table 5 below), TSB medium (Table 6 below), or MRS medium. The genera Bacillus and Micrococcus were cultured with shaking, while other strains were cultured in a stationary manner. *Bacillus coagulans,* Listeria, Pediococcus, and Enterococcus were cultured at 37°C, the other strains at 30°C.

**[Table 4]**

| Strain | Medium/culture temperature (°C) | Culture method |
|---|---|---|
| *Bacillus coagulans* JCM2257 | TSBYE/37 | Shaking |
| *Bacillus subtilis* JCM1465T | TSBYE/30 | Shaking |
| *Bacillus subtilis* IAM1381 | TSBYE/30 | Shaking |
| *Bacillus circulans* JCM2504T | TSBYE/30 | Shaking |
| *Micrococcus luteus* IFO12708 | TSBYE/30 | Shaking |
| *Listeria innocua* ATCC33090T | TSBYE/37 | Stationary |
| *Pediococcus pentosaceus* JCM5 885 | MRS/37 | Stationary |
| *Enterococcus faecalis* JCM5803T | MRS/37 | Stationary |
| *Enterococcus faecium* JCM5804T | MRS/37 | Stationary |
| *Lactococcus lactis* subsp. lactis ATTC19435 | MRS/30 | Stationary |
| *Lactobacillus plantarum* ATCC14917T | MRS/30 | Stationary |
| *Lactobacillus sakei* subsp. sakei JCM1157T | MRS/30 | Stationary |
| *Leuconostoc mesenteroides* subsp. mesenteroides JCM6124T | MRS/30 | Stationary |
| *Lactobacillus kimchii* JCM10707T | MRS/30 | Stationary |

[Table 5]

**Table 5: TSBYE Medium Composition**

| TSBYE medium composition | |
|---|---|
| TSB medium | 30.0 g/L |
| Yeast extract (Difco) | 6.0 g/L |

**[Table 6] TSB Medium Composition**

| Bacto Tryptic Soy Broth (TSB) medium composition (Difco) | |
|---|---|
| Pancreatic digest of casein | 17.0 g/L |
| Enzymatic digest of soybean meal | 3.0 g/L |
| Dextrose | 2.5 g/L |
| Sodium chloride | 5.0 g/L |
| Dipotassium monohydrogen phosphate | 2.5 g/L |

Antibiotic activity plates were also prepared. A 10 mL quantity of MRS agar medium (1.2 percent agar) and 5 mL of Lactobacilli AOAC agar medium (1.2 percent agar) were separately sterilized by heating to 121°C for 15 min and maintained at 55°C. The sterilized MRS agar medium was plated on a sterile Petri dish and placed on a clean bench for one hour. Next, 50 µL of indicator strain culture solution was admixed with Lactobacilli AOAC agar medium maintained at 55°C and layered onto the MRS plate. The plate was placed in a clean bench with the plate cover off for 15 minutes to dry out the surface.

The antibiotically active samples prepared above were added dropwise in increments of 10 µL, the covers were put on the plate, and drying was conducted for about an hour. The plates were cultured for 20 hours at the culture temperatures of the various indicator strains and the formation of growth inhibiting zones was examined. Antibiotic activity (AU/mL) was defined as follows. Antibiotic activity (AU/mL) = (maximum dilution ratio at which inhibition zones formed) x 1,000/10.

The antibiotic spectra of samples which were analyzed in this manner, and were found to be resistant to protease and exhibited broad antibiotic spectra.

The bacteriological properties of lactic acid bacterium strain AJ110263 selected by the above-described method were examined. This revealed homology of 98.22 percent (Table 7) with *Weissella confusa* strain ATCC10881 by 16S ribosome DNA (rDNA) base sequence homology analysis (Altshul, S.F., Madden, T.F., Schaffer, A.A., Zhang, J., Zhang, Z., Miller, W., and Lipman, D.J. (1997), Gapped BLAST and PSI-BLAST: a new generation of protein database search programs. Nucleic Acids Res. 25:3389-3402). Type cultures on deposit at the ATCC were employed in homology evaluation.

[Table 7]

**Table 7**

| | | |
|---|---|---|
| Homology of 16SrDNA | *Weissella confusa* | 98.22 % |
| | *Weissella viridescens* | 95.20 % |
| | *Weissella minor* | 92.54 % |
| | *Weissella kandleri* | 92.01 % |
| | *Weissella halotolerans* | 87.39 % |
| | *Weissella paramesenteroides* | 86.25 % |
| | *Lactobacillus mali* | 78.17 % |
| | *Pediococcus parvulus* | 77.58 % |

The basic characteristics (Table 8) of strain AJ110263 matched those of a lactic acid bacterium. Sugar fermentation (sugar consumption, see Table 9) was thought to be similar to fermentation by *Weissella confusa.* However, since L-arabinose fermentation differed and less than 100 percent homology with 16SrDNA was found, this new strain was found to be clearly different from known strains. This bacterium was named *Weissella sp.* AJ110263. This bacterium was deposited at the International Patent Organism Depositary of the National Institute of Advanced Industrial Science and Technology (an Independent Administration Institution). The accession number is FERM ABP-10474, as stated above.

[Table 8]

**Table 8: Basic characteristics of lactic acid bacterial strain AJ110263**

| | |
|---|---|
| Cell form | Short bacillus (0.8 to 1.0 x 1.0 to 1.5 µm) |
| Gram staining | (+) |
| Blast | (-) |
| Mobility | (-) |
| Colony form (medium: MRS medium) (medium temp.: 30°C) (culture period: 24 hr) | Round Smooth around edge Slightly protruding shape Some luster Milk-white |
| Culture temp. (37°C/45°C) | (+/-) |
| Catalase | (-) |
| Acid/gas production (glucose) | (+/-) |
| O/F test (glucose) | (+/+) |
| growth at pH 9.6 | (+) |
| growth in NaOH (6.5 percent) | (+) |

[Table 9]

**Table 9: Sugar fermentation properties of lactic acid bacterium strain AJ110263**

| Sugar fermentation properties | | |
|---|---|---|
| Fermentation (+) | L-arabinose | Albutin |
| | D-xylose | Esculin |
| | Galactose | Salicin |
| | Glucose | Cellobiose |
| | Fructose | Maltose |
| | Mannose | Cane sugar |
| | N-acetyl glucosamine | Gentiobiose |
| | Amygdalin | Gluconate |
| Fermentation (-) | Glycerol | Trehalose |
| | Erythritol | Inulin |
| | D-arabinose | Melezitose |
| | Ribose | Raffinose |
| | L-xylose | Starch |
| | Adonitol | Glucogen |
| | β-Methyl-D-xylose | Xylitol |
| | Sorbose | D-Thranose |
| | Rhamnose | D-Lyxose |
| | Dulcitol | D-Tagatose |
| | Inositol | D-Fucose |
| | Mannitol | L-Fucose |
| | Sorbitol | D-Arabitol |
| | α-Methyl-D-mannose | L-Arabitol |
| | α-Methyl-D-glucose | 2-Ketogluconic acid |
| | Lactose | 5-Ketogluconic acid |
| | Melibiose | |

### <Example 1>

The lactic acid bacterium *Weissella sp.* AJ110263 (FERM ABP-10474), isolated from fermented milk matsoon, and the lactic acid bacteria Pediococcus pentosaceus JCM5885, *Pediococcus pentosaceus* JCM5890, *Lactobacillus plantarum* JCM1149, and *Lactobacillus salivarius* JCM1231, obtained from type cultures, were precultured and cultured on MRS liquid medium (Table 1 above). The culture temperature was 30°C for *Weissella sp.* and 37°C for the other strains. The above lactic acid bacteria were inoculated onto and cultured for 24 hours on MRS agar medium plates to which "Umamizyme G", a protease derived from Aspergillus, had been added in quantities of 0 U/mL (none added), 200 U/ML, and 400 U/mL. In culturing, 100 mL of MRS medium was added to a 500 mL Sakaguchi flask, 100 mL of the above culture solution was inoculated, and the medium was shaken 100 times/min.

Next, *Lactobacillus sakei* strain JCM1157, which does not produce bacteriocin, was employed as an indicator strain and Lactobacilli AOAC medium was layered. These plates were cultured for 24 hours at 30°C, resulting in the formation of growth inhibition zones in the indicator bacteria (Table 10 below). From these results, each of the strains is shown to be producing protease-resistant bacteriocin.

[Table 10]

**Table 10**

| Strain | protease (U/mL) | | |
|---|---|---|---|
| | 0 | 200 | 400 |
| *Weissella sp.* AJ110263 | 7 | 10 | 10 |
| *Pediococcus pentosaceus* JCM5885 | 15 | 15 | 15 |
| *Pediococcus pentosaceus* JCM5890 | 10 | 12 | 12 |
| *Lactobacillus plantarum* JCM1149 | 15 | 17 | 23 |
| *Lactobacillus salivarius* JCM1231 | 13 | 18 | 18 |

*Lactobacillus sakei* JCM1157 was employed as indicator strain. The values in the table indicate the diameters of the growth inhibition zones.

### <Example 2>

*Lactococcus lactis* NCD0497 (a bacterium producing nisin A) and *Lactococcus lactis* NCIMB702054 (a bacterium producing nisin Z) were separately cultured in MRS liquid medium at 30°C. In the same manner as in example 1, antibiotic evaluation was conducted using *Lactobacillus sakei* strain JCM1157 as an indicator bacterium. Instead of nisin-producing bacterial strains, "nisin A 1,000 IU/mL solution" made by ICN Biomedical was spotted on MRS agar medium plates and the above antibiotic evaluation was conducted (without using bacterial strains).

Although indicator bacteria growth inhibition zones formed in the absence of protease, antibiotic activity due to nisin decreased as the protease concentration rose in the presence of protease (see Table 11).

**[Table 11]**

| Strain | protease (U/mL) | | |
|---|---|---|---|
| | 0 | 200 | 400 |
| Nisin A added (not containing any bacteria producing bacteriocin) | 30 | ND | ND |
| *Lactococcus lactis* NCD0497 (a bacterium producing nisin A) | 30 | 13 | ND |
| *Lactococcus lactis* NCIMB702054 (a bacterium producing nisin Z) | 30 | 13 | ND |

| | | | |
|---|---|---|---|
| *Lactobacillus sakei* JCM1157 was employed as indicator strain. The values in the table indicate the diameters of the growth inhibition zones. | | | |
| ND = not detected | | | |

### <Example 3>

Indicator bacteria *Weissella sp.* AJ110263 (FERM ABP-10474), *Pediococcus pentosaceus* JCM5885, *Lactococcus lactis* NCD0497 (a bacterium producing nisin A), and *Lactobacillus sakei* strain JCM 1157 were separately cultured and the culture solutions were centrifugally separated for 10 minutes at 10,000 rpm to obtain culture supernatants. After adding 200 U/mL of "Umamizyme G" to the culture supernatants and conducting protease treatment for 24 hours, the supernatants were filtered through 0.45 µm cellulose acetate ("DISMIC25CS" made by ADVANTEC) to obtain sterile samples. The spot-on-lawn method was employed to examine the antibiotic spectra. This revealed that *Weissella sp.* AJ110263 (FERM ABP-10474) and *Pediococcus pentosaceus* JCM5885 exhibited antibiotic activity even when treated with protease, as opposed to the nisin producing bacteria culture solution and *Lactobacillus sakei* JCM1157, which does not produce bacteriocin (see Table 12). Table 12 denotes the indicator bacteria as indicator strains and the lactic acid bacteria of the present invention as sample strains. It was thus understood that *Weissella sp.* AJ110263 (FERM ABP-10474) and *Pediococcus pentosaceus* JCM5885 produced protease-resistant bacteriocin.

[Table 12]

**Table 12**

| \Sample strains Indicator strains\ | *Weissella sp.* AJ 110263 | *Pediococcus pentosaceus* JCM5885 | *Lactococcus lactis* NCD0497 | *Lactobacillus sakei* JCM1157T |
|---|---|---|---|---|
| *Listeria innocua* ATCC33090T | 50 | 50 | ND | ND |
| *Bacillus circulags* JCM2504T | 100 | 100 | 50 | 50 |
| *Bacillus coagulans* JCM2257 | 50 | 100 | ND | ND |
| *Micrococcus lutens* IFO12708 | 100 | 100 | ND | ND |
| *Bacillus subtilis* JCM 1465T | 100 | 100 | ND | 50 |
| *Lactococcus lactis* subsp. Lactis ATCC19435 | 50 | 50 | ND | ND |
| *Enterococcus faecium* JCM5804T | 50 | 100 | ND | ND |
| *Enterococcus faecalis* JCM5809T | 100 | 100 | ND | 50 |
| *Lactobacillus plantarum* ATCC14917T | 100 | 100 | ND | 50 |
| *Lactobacillus sakei* JCM1157T | 50 | 50 | ND | ND |

| | | | | |
|---|---|---|---|---|
| After treating the culture solution with protease, antibiotic activity was measured by the spot-on-lawn method using the culture supernatant. The values indicate antibiotic activity. Antibiotic activity (AU/mL) = Maximum dilution ratio at which inhibition circles formed x 1,000/10, ND = not detected. | | | | |

### <Example 4>

Culture solutions of the various lactic acid bacteria shown in Table 13 - *Weissella sp.* AJ110263 (FERM ABP-10474), *Pediococcus pentosaceus* JCM5885, *Lactobacillus plantarum* JCM1149, *Lactobacillus salivarius* JCM1231, *Leuconostoc citreum* JCM9698, *Leuconostoc pseudomesenteroides* JCM9696 and JCM11045, *Lactococcus lactis* NCIMB702054 (a bacterium producing nisin Z) - were enzymatically treated in the same manner as in example 3 and antibiotic activity was measured by the spot-on-lawn method using *Bacillus subtilis* as indicator bacterium. In the same manner as in example 3, "Umamizyme G" a protease derived from *Aspergillus oryzae* was employed. Furthermore, 100 units/mL of α-amylase derived from *Bacillus subtilis* (Wako Junyaku) was added to the culture solutions of the lactic acid bacteria and reacted for more than an hour at 30°C. Similarly, employing *Bacillus subtilis* IAM1381 as the indicator bacterium, antibiotic activity was measured by the spot-on-lawn method to check the effect of α-amylase on antibiotic activity.

[Table 13]

**Table 13**

| Strain | | Residual antibiotic activity | | |
|---|---|---|---|---|
| | | Control | Umamizyme | α-Amylase |
| Nisin producer | | | | |
| | *Lactococcus lactis* NCIMB702054 | 100 | nd | 100 |
| PRB producers | | | | |
| | *Weissella sp.* AJ110263 | 100 | 100 | 30 |
| | *Weissella cibaria* JCM 12495 | 100 | 100 | 30 |
| | *Weissella confusa* JCM1093 | 100 | 70 | 50 |
| | *Weissella hellenica* JCM 10103 | 100 | 100 | 40 |
| | *Weissella kandleri* JCM5817 | 100 | 100 | 40 |
| | *Weissella minor* JCM1168 | 100 | 100 | 40 |
| | *Weissella paramesenteroides* | 100 | 100 | 70 |
| JCM9890 | | | | |
| | *Weissella thailandensis* JCM10694 | 100 | 100 | 40 |
| | *Pediococcus pentosaceus* JCM5885 | 100 | 90 | 30 |
| | *Lactobacillus plantarum* JCM1149 | 100 | 80 | 30 |
| | *Lactobacillus salivarius* JCM1231 | 100 | 80 | 30 |
| | *Lactobacillus pentosus* IAM1558 | 100 | 100 | 30 |
| | *Leuconostoc citreum* JCM9698 | 100 | 80 | 40 |
| | *Leuconostoc pseudomesenteroides* | 100 | 100 | 50 |
| JCM9696 | | | | |
| | *Leuconostoc pseudomesenteroides* | 100 | 100 | 50 |
| JCM11045 | | | | |
| | *Leuconostoc argentinum* JCM11052 | 100 | 100 | nd |
| | *Leuconostoc carnosum* JCM 9695 | 100 | 100 | 30 |
| | *Leuconostoc mesenteroides* JCM6124 | 100 | 100 | 40 |

As shown in Fig. 13, culture solutions of *Weissella sp.* AJ110263 (FERM ABP-10474), *Weissella cibaria* JCM12495, *Weissella confusa* JCM1093, Weissella hellenica JCM10103, *Weissella kandleri* JCM5817, *Weissella minor* JCM1168, *Weissella paramesenteroides* JCM9890, *Weissella thailandensis* JCM10694, *Pediococcus pentosaceus* JCM5885, *Lactobacillus plantarum* JCM1149, *Lactobacillus salvarius* JCM1231, *Lactobacillus pentosus* JCM1558, *Leuconostoc citreum* JCM9698, *Leuconostoc pseudomesenteroides* JCM9696 and JCM11045, *Leuconostoc argentinum* JCM11052, *Leuconostoc carnosum* JCM9695, and *Leuconostoc mesenteroides* JCM6124 exhibited antibiotic activity even when treated with protease. Thus, each of these strains was determined to produce protease-resistant bacteriocin. It was confirmed that the antibiotic activity of these culture solutions was confirmed to decrease when treated with α-amylase. The residual activity of Table 13 was calculated as follows: Antibiotic activity (AU/mL) = maximum dilution ratio at which inhibition zones formed x 1,000/10 x (inhibition circle diameter of enzyme treatment sample/diameter of inhibition circle of control).

### <Example 5>

### <Measurement of antibiotic activity in rumen juice>

*Lactococcus lactis* NCIMB8780 (a bacterium producing nisin A), *Lactococcus lactis* NCIMB702054 (a bacterium producing nisin Z), *Weissella sp.* AJ110263 (a bacterium producing PRB), and *Lactococcus lactis* ATCC19435 (a non-bacteriocin-producing bacterium) were cultured in stationary fashion for 24 hours at 30°C in MRS medium.

### (a) Rumen juice treatment

The culture solutions of the lactic acid bacteria were centrifugally separated at 12,000 rpm and filtered through a 0.45 µm cellulose acetate filter ("DISMIC25CS" made by ADVANTEC) to prepare sterile supernatants. A 1 mL quantity of rumen juice was added for each 4 mL of culture supernatant and the mixture was reacted for 24 hours at 37°C. The reaction solution was adjusted to pH 5.5 with NaOH and hydrochloric acid and concentrated fourfold at 30°C with a vacuum concentrator.

### (b) Antibiotic activity test

A 50 µL quantity of indicator bacterium in the form of *Bacillus subtilis* was spread on a "GAM" agar medium (Nissui Pharmaceutical Co., Ltd.) plate. After thoroughly drying the surface of the plate, the prepared sample of paragraph (a) above was applied in 10 µL, spots and cultured for 24 hours at 30°C. The formation of inhibition zones was subsequently confirmed. The "+" signs in Table 14 below denote the size of the diameter of the inhibition zones. When treated with rumen juice, nisin lost its antibiotic activity, but PRB maintained it. In greater detail, in Table 14, a "+" indicates antibiotic activity, a "-" indicates no antibiotic activity. When used to represent the diameter of growth inhibition zones, "++++" denotes 10 mm, "+++" denotes 7 mm, and "++" denotes 5 mm.

[Table 14]

**Table 14: Evaluation of the stability of antibiotic activity in rumen**

| Strains (Bacteriocin) | rumen juice | |
|---|---|---|
| | (-) | (+) |
| Control (rumen juice) 10% | | |
| *Lactococcus lactis* AJ13981 (non bacteriocin producer) | - | |
| *Lactococcus lactis* NCIMB8780 (nisin A producer) | +++ | |
| *Lactococcus lactis* NCIMB702054 (nisin Z producer) | ++++ | |
| *Weissella sp* AJ110263 (PRB producer) | +++ | ++ |

### <Example 6>

### <A test of the methane production inhibiting effect in the rumen>

### (a) Culturing of lactic acid bacteria

Supernatants 1 to 4 were prepared as shown in Table 15. *Lactococcus lactis* NCIMB702054 (a strain producing nisin Z) was cultured for 20 hours at 30°C at pH 5.5 (NaOH) in medium containing 0.81 % yeast extract, 0.5 % sodium chloride, and 2.2 % glucose. The culture solution was centrifugally separated for 10 minutes at 10,000 rpm and filtered through a 0.45 µm cellulose acetate filter ("DISMIC25CS" made by ADVANTEC) to prepare sterile supernatant 1. To confirm the effect of nisin, 40 units/mL of "Umamizyme G" (Amano Enzymes) was added to supernatant 1 and a nisin-degraded supernatant solution 2 was prepared.

*Weissella sp.* AJ110263 (a strain producing protease-resistant bacteriocin) was cultured for 24 hours at 30°C in MRS medium to which 0.1 percent L-Cys and 0.1 percent L-Met had been added. The culture solution was centrifugally separated in the same manner as above and filtered to prepare sterile supernatant 3. To confirm the effect of this culture solution, *Lactococcus lactis* NCIMB702054 was cultured in the same medium and a sterile supernatant was prepared. "Umamizyme G" (Amano Enzymes) was added to prepare nisin degraded supernatant 4.
Table 15 shows the components of the culture supernatant.

[Table 15]

**Table [15]**

| Supernatant | Sample | Strain | Medium | Protease | Glc(%) | Lac(%) |
|---|---|---|---|---|---|---|
| 1 | Nisin | *Lactococcus lactis* | Glc+YE | - | 0.4 | 2.3 |
| 2 | Control-1 (nisin) | *Lactococcus lactis* | Glc+YE | + | 0.4 | 2.3 |
| 3 | PRB | *Weissella sp.* (Ws) | MRS | - | 0.9 | 1.3 |
| 4 | Control-2 (PRB) | *Lactococcus lactis* | MRS | + | 1.1 | 1.3 |

| | | | | | | |
|---|---|---|---|---|---|---|
| Glc (%) = glucose consumption level Lac (%) = lactic acid production level YE = yeast extract | | | | | | |

### (b) Confirmation of the effect on methane production inhibition in the rumen

*In vitro* rumen juice to which 10 percent of the culture supernatant described in paragraph (a) above had been added was cultivated for 48 hours in a bioreactor system for the analysis of continuous methane production.

### <Materials and Test Equipment>

Testing was conducted with an in vitro continuous culture system (Takasugi Seiyakusho) comprised of four 1,000 mL fermentation vat containers equipped with buffer solution, solid feed administration inlet, thermistor probe, nitrogen gas administration inlet, magnetic stirrer, near infrared methane and carbon dioxide analyzer. Two non-lactating Holstein cows (average weight 800 kg) were employed as donors. With a surgical procedure developed on animals at the facilities of the Obihiro University of Agriculture and Veterinary Medicine and approved by committee, fistula were created in the rumens and the cows were kept in a tie stall barn. While keeping a basic feed of hay (DM: 87.33 %; OM: 98.98 %; CP: 14 %; ADF: 38.84 %; NDF: 73.26 %; ADL: 4.10 %; GE: 4.45 Mcal) at maintenance level (55 g DM kg - 0.75 BW/day), equal amounts were fed twice daily at 8:00 am and 5:00 pm. The animals had free access to water and sodium chloride blocks (Fe: 1232, Cu: 150, Co: 25, Zn: 500, I: 50, Se: 15, and Na: 382 mg/kg). Rumen juice was collected from the cows before the morning feeding, immediately strained with a nylon bag, and placed in a triangular flask with an open mouth permitting the entry of oxygen. The strained rumen juice (400 mL) of one of the cows was admixed with an autoclaved buffer solution (400 mL) 11). All of the cultures were conducted four times anaerobically at 39°C for 24 hours with 10 g of the above described powder feed (1 mm screen) added as matrix. In each test, two containers were randomly allocated. To create an anaerobic state, nitrogen was introduced at a rate of 20 mL/min. Using the above-described culture fermentation base unit (Takasugi Seisakusho K.K., Tokyo), the temperature of the fermentation containers was maintained at 39°C and the contents were mixed.

### <Method of analyzing methane and carbon dioxide>

All the prescribed materials were introduced into an in vitro rumen system and the reaction was started. Denoting the start of the reaction as time 0, rumen samples were collected at 0, 0.5, 1, 1.5, 2, 4, 6, 8, 10, 12, and 24 hours and the pH and cell growth (OD 660) were immediately checked. The pH of the rumen juice was measured with a ph meter (HM-21P, Toa Denpa Kogyo, Tokyo). Before measuring the OD 660 with a spectrophotometer (Part No. 100-004, Serial No. 5667-15, Hitachi, Tokyo), the samples were diluted 1:200 with distilled water. The continuous methane and carbon dioxide production rates were measured automatically with an infrared gas analyzer (Takasugi Seisakusho). Next, the data were sent from the analyzer through an interface at one minute intervals to a computer (Windows (registered trademark) XP Professional 1-2 CPU, 1MB Corporation) and stored.

### <Statistical analysis method>

Continuous in vitro culturing was repeated twice a day for two days (n = 4) for each test. Cumulative methane and carbon dioxide production over 24 hours was inferred by Bertalanffy model nonlinear regression analysis (methane (mL) or carbon dioxide (mL) = a + b (1- e - ct)3, where *a* denotes initial methane or carbon dioxide production, b denotes second methane or carbon dioxide production, c denotes a certain methane and carbon dioxide production rate, and *t* denotes time (min)). Differences in the average values of the test results were analyzed by one-way layout dispersion analysis using the General Linear Model Procedures of the Statistical Analysis Systems Institute (SAS, 1994). Statistically significant differences between average values in individual test zones were compared to Duncan's multiple-range test. The statistically significant level was set at P < 0.05.

Continuous in vitro culturing was repeated twice a day for two days (n = 4) for each test. Cumulative methane and carbon dioxide production over 24 hours was inferred by Bertalanffy model nonlinear regression analysis (methane (mL) or carbon dioxide (mL) = a + b (1- e - ct)3, where a denotes initial methane or carbon dioxide production, b denotes second methane or carbon dioxide production, c denotes a certain methane and carbon dioxide production rate, and *t* denotes time (min)). The average values of the test results were analyzed by one-way layout dispersion analysis using the General Linear Model Procedures of the Statistical Analysis Systems Institute (SAS, 1994). Test averages were statistically compared to Duncan's multiple-range test. The statistically significant level was set at P < 0.05.
Table 16 shows the amount of methane (CH₄) and carbon dioxide (CO₂) produced per minute. PRB derived from Weissella clearly suppressed the production of CH₄ and CO₂ better than nisin.

[Table 16]

**Table [16]**

| Sample | CH₄ (mL/min) | CO₂ (mL/min) |
|---|---|---|
| 1 Nisin | 0.16 | 0.55 |
| 2 Control-1 (nisin) | 0.14 | 0.40 |
| 3 PRB | 0.16 | 0.49 |
| 4 Control-2 (PRB) | 0.19 | 0.57 |

### <Example7>

### <Nisin stability test in rumen>

*Lactococcus lactis* AJ13981 (ATCC19435), a strain that does not produce nisin, was cultured for 24 hours at 30°C in MRS medium. The culture solution was centrifugally separated for 10 min at 10,000 rpm and filtered through a 0.45 µm cellulose acetate filter (ADVANTEC "DISMIC25CS") to prepare sterile supernatant. A 10 percent quantity of this culture supernatant and 10 µM of the reagent nisin A were added to the in vitro rumen juice and cultured for 48 hours with a bioreactor system to analyze continuous methane production. (The molecular weight of nisin is 3,400 Daltons, corresponding to about 40 IU per µg. Accordingly, 10 µM of nisin corresponded to 1,360 IU/mL). As shown in Table 17 below, supernatant was prepared as set forth above and the nisin concentration was measured by HPLC and a bioassay method. In the bioassay method, the method of Embodiment 5 was employed with *Lactobacillus sakei* JCM1157 serving as the indicator bacterium. As indicated in Table 17, the nisin in the rumen samples lost its antibiotic activity about one hour after addition, with antibiotic activity being nearly completely eliminated within [at most] six hours of addition.

[Table 17]

**Table [17]**

| Culture time (hr) | Nisin A (IU/mL) | |
|---|---|---|
| | HPLC | Bioassay |
| Initial concentration | 1360 | - |
| 0.1 | 350 | 30 |
| 0.5 | 260 | 20 |
| 1.0 | 150 | 10 |
| 2.0 | 110 | nd |
| 4.0 | 90 | nd |
| 6.0 | nd | nd |
| 12.0 | nd | nd |
| 24.0 | nd | nd |

| | | |
|---|---|---|
| 10 µM = 1,360 IU/mL | | |
| The MW of nisin is 3,400, 1 µg = 40 IU | | |

## Claims

1. A methane production reducer for ruminants comprising protease-resistant bacteriocin isolated from a lactic acid bacterium.

2. The methane production reducer of claim 1, wherein protease-resistant bacteriocin is from a lactic acid bacteria culture solution and/or lactic acid culture supernatant.

3. The methane production reducer of claim 2, wherein said protease-resistant bacteriocin is formulated for administration to ruminants.

4. The methane production reducer of claim 1 to 3, wherein said lactic acid bacterium is one or more lactic acid bacteria selected from the group consisting of the bacterium belonging to genus *Lactobacillus, Weissella, Pediococcus,* and *Leuconostoc.*

5. The methane production reducer of claim 4, wherein said the genus *lactobacillus* is one or more lactic acid bacteria selected from the group consisting of *Lactobacillus plantarum, Lactobacillus salivarius,* and/or *Lactobacillus pentosus.*

6. The methane production reducer of claim 4, wherein the genus *Weissella* is one or more lactic acid bacteria selected from the group consisting of *Weissella sp.* FERM ABP-10474, *Weissella cibaria, Weissella confusa, Weissella hellenica, Weissella kandleri, Weissella minor, Weissella paramesenteroides,* and/or *Weissella thailandensis.*

7. The methane production reducer of claim 4, wherein the genus *Pediococcus* is *Pediococcus pentosaceus.*

8. The methane production reducer of claim 4, wherein the genus *Leuconostoc* is one or more lactic acid bacteria selected from the group consisting of *Leuconostoc citreum, Leuconostoc pseudomesenteroides, Leuconostoc argentinum, Leuconostoc carnosum,* and/or *Leuconostoc mesenteroides.*

9. A feed composition for ruminants comprising the methane production reducer of claims 1 to 8.

10. A method of improving the growth of ruminant by administering the feed composition of claim 9 to said ruminant.

11. A use of a methane production reducer for ruminants comprising protease-resistant bacteriocin isolated from a lactic acid bacterium.

12. The use of claim 11, wherein said lactic acid bacterium is one or more lactic acid bacteria selected from the group consisting of the genus *Lactobacillus, Weissella, Pediococcus,* and *Leuconostoc.*

13. The use of claim 12, wherein said the genus *Lactobacillus* is one or more lactic acid bacteria selected from the group consisting of *Lactobacillus plantarum, Lactobacillus salivarius,* and/or *Lactobacillus pentosus.*

14. The use of claim 12, wherein the genus Weissella is one or more lactic acid bacteria selected from the group consisting of *Weissella sp.* FERM ABP-10474, *Weissella cibaria, Weissella confusa, Weissella hellenica, Weissella kandleri, Weissella minor, Weissella paramesenteroides,* and/or *Weissella thailandensis.*

15. The use of claim 12, wherein the genus *Pediococcus* is *Pediococcus pentosaceus.*

16. The use of claim 12, wherein the genus *Leuconostoc* is one or more lactic acid bacteria selected from the group consisting of *Leuconostoc citreum, Leuconostoc pseudomesenteroides, Leuconostoc argentinum, Leuconostoc carnosum,* and/or *Leuconostoc mesenteroides.*

17. The use of a feed composition for ruminants comprising the methane production reducer of claims 1 to 8.
